Europäisches Patentamt

European Patent Office

Office européen des brevets

(11) Numéro de publication : **0 471 595 A1**

# (12) DEMANDE DE BREVET EUROPEEN

(21) Numéro de dépôt : **91401806.4**

(22) Date de dépôt : **02.07.91**

(51) Int. Cl.⁵ : **C08K 3/36,** C08F 2/44, C08F 20/26, A61L 15/00, C08L 33/02

(30) Priorité : **14.08.90 FR 9010338**

(43) Date de publication de la demande :
**19.02.92 Bulletin 92/08**

(84) Etats contractants désignés :
**AT BE CH DE ES FR GB IT LI LU NL SE**

(71) Demandeur : **SOCIETE FRANCAISE HOECHST TOUR ROUSSEL HOECHST 1 Terrasse Bellini F-92800 Puteaux (FR)**

(72) Inventeur : **Mallo, Paul**
**9, Boulevard de la République**
**F-78400 Chatou (FR)**

(74) Mandataire : **Rinuy, Santarelli**
**14, avenue de la Grande Armée**
**F-75017 Paris (FR)**

(54) **Nouveaux superabsorbants en poudre, contenant de la silice, leur procédé de préparation et leur application.**

(57) Superabsorbants en poudre, hydrophiles, insolubles dans l'eau, contenant pondéralement, à l'état sec, en combinaison, de 1 à 45% de silice colloïdale à l'état de particules non agglomérées d'un diamètre moyen compris entre 9 et 50 nm et de 99 à 55% d'un polymère réticulé à base d'acide acrylique libre, partiellement ou totalement salifié par du sodium ou du potassium, procédé de préparation et application notamment à l'absorption des fluides aqueux.

EP 0 471 595 A1

Jouve, 18, rue Saint-Denis, 75001 PARIS

Dans le domaine des superabsorbants, désignés ci-après SAP, on utilise couramment des polymères en poudre, réticulés, hydrophiles, insolubles dans l'eau, à base d'acide acrylique libre, partiellement ou totalement salifié par un métal alcalin.

Ces polymères sont généralement obtenus par broyage de gels polymères hachés et séchés, ces gels polymères étant issus de réaction de polymérisation en solution aqueuse d'acide acrylique libre, partiellement ou totalement salifié par un métal alcalin. On sait que ces réactions de polymérisation en solution aqueuse sont réalisées de façon quasi adiabatique au départ de solutions aqueuses à faibles concentrations en monomères (de 10 à 25 % environ) de manière à pouvoir contrôler la réaction exothermique. En fin de polymérisation, on obtient des gels polymères gonflés avec toute l'eau utilisée dans la réaction de polymérisation qui doivent être séchés afin de pouvoir les transformer en poudre.

La manipulation de ces gels gonflés d'eau est laborieuse et délicate car ils sont mous et visqueux, et leur séchage puis leur broyage sont d'autant plus onéreux qu'ils contiennent plus d'eau.

Afin d'obvier à ces inconvénients, la demanderesse a découvert de nouveaux superabsorbants, en poudre, réticulés, hydrophiles, insolubles dans l'eau, à base de silice et de polymères d'acide acrylique libre, partiellement ou totalement salifié par un métal alcalin.

Dans le cadre de la présente invention, on désigne par métal alcalin le sodium ou le potassium et par silice, de la silice colloïdale, à l'état de particules élémentaires non-agglomérées, d'un diamètre moyen compris entre 9 et 50 nanomètres.

Les produits selon la présente invention sont caractérisés par le fait qu'ils contiennent pondéralement, à l'état sec, et en combinaison, de 1 à 45 % de silice et de 99 à 55 % d'un polymère réticulé à base d'acide acrylique libre partiellement ou totalement salifié par un métal alcalin, de préférence le sodium ou le potassium.

L'invention a notamment pour objet les produits tels que définis ci-dessus caractérisés en ce qu'ils contiennent pondéralement, à l'état sec, et en combinaison, de 10 à 40 % de silice et de 90 à 60 % d'un polymère réticulé acide acrylique-acrylate de métal alcalin, de préférence le sodium ou le potassium, contenant en proportions molaires de 10 à 50 % de motifs acide acrylique.

Parmi ces derniers produits, l'invention a plus particulièrement pour objet les produits tels que définis ci-dessus caractérisés en ce qu'ils contiennent pondéralement, à l'état sec et en combinaison, de 10 à 40 % de silice colloïdale dont les particules ont un diamètre moyen de 20 à 50 nm et de 90 à 60 % d'un copolymère réticulé acide acrylique-acrylate de sodium ou de potassium contenant en proportions molaires de 15 à 35 % de motifs acide acrylique.

Les polymères présents dans les produits de la présente invention sont réticulés de préférence avec un monomère ou plusieurs monomères diéthyléniques solubles dans l'eau tels que l'acide bisacrylamidoacétique ou l'acide diallyloxyacétique ou l'un de leurs sels de métal alcalin ou d'ammonium. La quantité de monomères réticulants utilisée peut varier dans de grandes proportions, mais habituellement, on utilise de 0,05 à 0,5 nmole de monomères réticulants par mole d'acide acrylique libre ou salifié mis en oeuvre.

Les produits de la présente invention sont en poudre, insolubles dans l'eau, hydrophiles et gonflables à l'eau. Ils contiennent peu ou pas de monomères résiduels. Leur capacité d'absorption d'eau, déterminée selon le test T1 décrit ci-après est supérieure à 200 g par g de produit sec; leur capacité d'absorption d'eau salée à 9 g de chlorure de sodium par litre, déterminée selon le test T2 décrit ci-après est supérieure à 25 g par g de produit sec.

Selon le test T1, on agite durant 30 minutes à 20°C, o,5 g de produit à tester dans 500 g d'eau, puis on pèse le gel obtenu après l'avoir laisser s'égoutter. Le poids trouvé est ramené à 1 g de produit sec. Selon le test T2, on agite durant 30 minutes à 20°C, 2 g de produit à tester dans 500 g d'une solution aqueuse de chlorure de sodium à 9 g par litre puis on pèse le gel obtenu après l'avoir laisser s'égoutter. Le poids trouvé est ramené à 1 g de produit sec.

Les produits de la présente invention ne sont pas un simple mélange de silice colloïdale et d'un polymère réticulé à base d'acide acrylique libre, partiellement ou totalement salifié par un métal alcalin, mais une véritable combinaison de la silice avec le polymère. Les gels gonflés d'eau obtenus avec les produits de l'invention sont fermes, non collants et aisément manipulables contrairement au gels gonflés d'eau obtenus avec les produits de l'art antérieur, qui sont mous et visqueux. Le fait d'obtenir avec les produits de l'invention des gels gonflés d'eau fermes et non collants est extrêmement intéressant d'un point de vue industriel et applicatif, car d'une part les produits de l'invention peuvent être aisément obtenus par un procédé de polymérisation en solution aqueuse, et d'autre part, les produits de l'invention présentent des capacités d'absorption d'eau et d'eau salée sensiblement identiques à celles présentées par les produits de l'art antérieur tout en contenant moins de matière organiques.

La présente invention concerne également un procédé d'obtention des produits définis ci-dessus caractérisé en ce que l'on polymérise dans l'eau, en mélange, la silice colloïdale et l'acide acrylique libre, partiellement ou totalement salifié par un métal alcalin, en présence d'un monomère réticulant hydrosoluble et d'un amorceur de polymérisation hydrosoluble, puis que l'on sèche le gel polymère gonflé d'eau ainsi obtenu jusqu'à une teneur en eau inférieure à 10 % en poids et qu'enfin on le broit en poudre.

Le procédé est réalisé de préférence en atmosphère inerte dans de l'eau contenant pondéralement de 1 à 20 % en poids de silice colloïdale et, à l'état dissous, de 5 à 25 % en poids d'acide acrylique libre, partiellement ou totalement salifié par un métal alcalin ainsi que la quantité désirée de monomère réticulant.

Les amorceurs de polymérisation utilisés sont les amorceurs hydrosolubles couramment utilisés pour la polymérisation en solution aqueuse de l'acide acrylique partiellement ou totalement salifié par un métal alcalin. On citera notamment les systèmes redox persulfate de métal alcalin ou d'ammonium - bisulfite ou disulfite ou sulfite de métal alcalin ou d'ammonium. Avantageusement, on utilisera le système redox persulfate de sodium - disulfite de sodium. Le monomère réticulant choisi sera de préférence l'acide bisacrylamidoacétique ou l'acide diallyloxyacétique.

La réaction de polymérisation est amorcée par l'introduction, lente et progressive dans le milieu réactionnel agité et soigneusement désoxygéné, d'une solution aqueuse de l'amorceur choisi. Lorsque le système amorceur est un couple redox, on introduit de préférence successivement dans un milieu réactionnel une solution aqueuse de chaque constituant de ce couple. La réaction de polymérisation est généralement amorcée à une température comprise entre 20 et 40°C, puis elle est de préférence conduite de manière quasi adiabatique. Dès l'amorçage, le milieu réactionnel se gélifie progressivement si bien qu'il ne peut plus être agité. En fin de réaction exothermique, on maintient le milieu réactionnel pendant 30 minutes à 3 heures, à une température de 40 à 60°C. On obtient ainsi un gel gonflé d'eau, non collant, de bonne tenue mécanique que l'on peut sortir du réacteur de polymérisation, puis sécher en étuve ventilée à une température inférieure à 80°C. Le rendement de la polymérisation est quantitatif et le produit isolé après séchage est une matière blanche, solide, friable, facilement réduite en poudre fine. Avantageusement, en sortie du réacteur de polymérisation, le gel est haché ou extrudé avant d'être séché, puis broyé. On obtient un produit sous forme d'une poudre blanche ayant la granulométrie désirée exempt de monomères résiduels et insoluble dans l'eau.

Les polymères selon l'invention présentent d'intéressantes propriétés absorbantes de fluides aqueux; ils manifestent notamment un fort pouvoir absorbant de solutions aqueuses contenant des sels minéraux hydrosolubles, aussi bien des sels de métaux alcalins qu'alcalino-terreux et ils sont aptes à absorber des quantités relativement importantes d'eau de mer. Ils trouvent aussi de remarquables applications dans la fabrication de certains articles d'hygiène, destinés notamment à la rétention d'urine.

les exemples suivants illustrent l'invention sans toutefois la limiter.

### Exemple 1

On prépare sous agitation et à la température ambiante, une phase aqueuse contenant :
- **108 g** (1,5 mole) d'acide acrylique,
- **0,325 g** (0,6 mmole) de diéthylènetriaminepentaacétate de sodium, (DTPANa),
- **0,0546 g** (0,276 mmole) d'acide bisacrylamidoacétique, (ABAA),
- **463,65 g** d'eau contenant en solution 60,6 g (1,08 mole) d'hydroxyde de potassium,
- **80 g** d'un sol de silice commercial contenant pondéralement 50% d'eau et 50% de silice colloïdale dont les particules non agglomérées ont un diamètre moyen de 50 nm.

Cette phase aqueuse est ensuite placée dans un réacteur de polymérisation, puis elle est soigneusement désoxygénée par un barbotage d'azote et enfin elle est chauffée à 40°C sous agitation, en atmosphère inerte.

A cette température, on introduit successivement, en 5 minutes, une solution de 0,08 g (0,336 mmole) de persulfate de sodium dissous dans 10 g d'eau, puis en 10 minutes une solution de 0,06 g (0,316 mmole) de disulfite de sodium dissous dans 10 g d'eau.

A ce stade, le poids de la phase aqueuse est d'environ 672 g et elle contient pondéralement 22,2 % de monomères, 5,9 % de silice et environ 71,8 % d'eau. Le taux de monomère réticulant est de 0,092 mmole pour une mole de monomère et l'amorçage de la réaction de polymérisation est réalisée avec 0,225 mmole de persulfate de sodium par mole de monomère et 0,210 mmole de disulfite de sodium par mole de monomère.

Après amorçage, il se développe aussitôt une réaction exothermique et le milieu réactionnel se gélifie progressivement. On arrête l'agitation et on abandonne le milieu réactionnel 3 heures à 40-45°C en atmosphère inerte.

Ensuite, après refroidissement du milieu réactionnel à 20°C, le gel polymère est isolé, haché et enfin séché en étuve ventilée à 80°C pendant 15 heures. Le produit obtenu est ensuite broyé en poudre fine.

On obtient ainsi 189 g d'une poudre blanche, contenant 5,2 % d'eau. Ce produit contient pondéralement à l'état sec, 21,15 % de silice et 78,85 % d'un copolymère réticulé acide acrylique - acrylate de potassium, 28-72 en proportions molaires.

Dans les tests T1 et T2, ce produit présente respectivement une capacité d'absorption d'eau de 248 g/g et une capacité d'absorption d'eau salée de 34 g/g.

Exemple 2 - 3

En opérant dans les mêmes conditions que dans l'exemple 1, au départ de 1,5 mole d'acide acrylique neutralisé à 72 % avec de l'hydroxyde de potassium, et en conservant une concentration de monomère de 22,2 % dans la phase aqueuse et les mêmes quantités en proportions molaires d'amorceur, on prépare les produits suivants, mentionnés dans le tableau I en faisant varier d'une part les quantités de silice, et d'autre part, les quantités de monomère réticulant.

Exemple 4

On prépare sous agitation et à la température ambiante, une phase aqueuse contenant :
– **108 g** (1,5 mole) d'acide acrylique,
– **0,325 g** (0,6 mmole) de diéthylènetriaminepentaacétate de sodium, (DTPNa),
– **0,0546 g** (0,276 mmole) d'acide bisacrylamidoacétique, (ABAA),
– **303 g** d'eau
– **80 g** d'un sol de silice commercial contenant pondéralement 50% d'eau et 50% de silice colloïdale dont les particules non agglomérées ont un diamètre moyen de 50 nm.

Cette phase aqueuse est ensuite placée dans un réacteur de polymérisation, puis elle est soigneusement désoxygénée par un barbotage d'azote et enfin elle est chauffée à 45°C sous agitation en atmosphère inerte.

A cette température, on introduit successivement, en 5 minutes, une solution de 0,08 g (0,336 mmole) de persulfate de sodium dissous dans 10 g d'eau, puis en 10 minutes une solution de 0,06 g (0,316 mmole) de disulfite de sodium dissous dans 10 g d'eau.

A ce stade, le poids de la phase aqueuse est d'environ 519 g et elle contient pondéralement 20,8 % de monomères, 7,7 % de silice et environ 71,5 % d'eau. Le taux de monomère réticulant est de 0,184 mmole pour une mole de monomère et l'amorçage de la réaction est réalisée avec 0,225 mmole de persulfate de sodium par mole de monomère et 0,0210 mmole de disulfite de sodium par mole de monomère.

Après amorçage, il se développe aussitôt une réaction exothermique et le milieu réactionnel se gélifie progressivement. On arrête l'agitation et on abandonne le milieu réactionnel 3 heures à 40-45°C en atmosphère inerte.

Ensuite, après refroidissement du milieu réactionnel à 20°C, le gel polymère est isolé et haché. Celui-ci est alors replacé dans un réacteur de polymérisation. On ajoute alors très lentement, et sous agitation une solution aqueuse de 60,6 g (1,08 mole) de potasse dissous dans 600 g d'eau. On laisse ensuite l'ensemble sous agitation pendant 8 heures de manière à assurer l'homogénéité de la neutralisation.

Le gel polymère est alors séché à 80 °C dans une étuve ventilée pendant 15 heures, puis broyé.

Dans les tests 1 et 2, ce produit présente une capacité d'absorption d'eau de 80 g/g et une capacité d'absorption d'eau salée de 40 g/g.

Exemple de comparaison A

On prépare comme dans l'exemple 1 une phase aqueuse contenant :
– **129,71 g** (1,8 mole) d'acide acrylique
– **0,39 g** (50,72 mmole) de DTPA, Na
– **0,0656 g** (0,332 mmole) d'ABAA,
– **701,8 g** d'eau contenant en solution 76,72 g (1,37 mole) d'hydroxyde de potassium.

Cette phase aqueuse est ensuite placée dans un réacteur de polymérisation, puis elle est soigneusement désoxygénée par un barbotage d'azote et enfin elle est chauffée à 40°C sous agitation, en atmosphère inerte. A cette température, on introduit successivement sous agitation, en 5 minutes, une solution de 0,096 g (0,403 mmole) de persulfate de sodium dans 10 g d'eau, puis en 10 minutes une solution de 0,072 g (0,379 mmole) de disulfite de sodium dans 10 g d'eau.

Le milieu réactionnel est ensuite traité comme dans l'exemple 1. On obtient ainsi un produit exempt de silice dont les caractéristiques sont données dans le tableau 1.

Exemple de comparaison B

On prépare sous agitation et à la température ambiante, une phase aqueuse contenant :
– **108 g** (1,5 mole) d'acide acrylique

– **0,325 g** (0,6 mole) de diéthylènetriaminepentaacétate de sodium (DTPA Na),
– **0,0273 g** (0,138 mmole) d'acide bisacrylamidoacétique (ABAA),
– **503,65 g** d'eau contenant en solution 60,6 g (1,08 mole) d'hydroxyde de potassium.

On ajoute ensuite 40 g de silice en poudre présentant un diamètre moyen des particules de 10 micromètres. On ne peut la disperser dans le milieu réactionnel malgré l'utilisation d'une agitation très énergique. Après 3 jours d'agitation énergique, la silice n'est toujours pas dispersée dans le milieu réactionnel. L'essai est alors abandonné.

Exemple de comparaison C

On mélange à sec, dans un mortier, 7,9 g de polymère tel que préparé à l'exemple de comparaison B avec 2,1 g de silice en poudre, présentant un diamètre moyen des particules de 10 micromètres.

Dans les tests 1 et 2, ce produit présente une capacité d'absorption d'eau de 138 g/g et une capacité d'absorption d'eau salée de 14 g/g.

## – TABLEAU I –

| Ex | Co | TxN | TxR | Cs | Pds | E.S. | $H_2O$ | T1 | T2 |
|----|------|-----|-----|------|-----|------|------|-----|------|
| 1 | 22,2 | 72 | 184 | 21,2 | 189 | 94,8 | 2,5 | 248 | 34 |
| 2 | 22,2 | 72 | 184 | 10 | 166 | 95,0 | 3,0 | 294 | 39 |
| 3 | 22,2 | 72 | 184 | 35,0 | 229 | 95,7 | 1,9 | 323 | 35,5 |
| 4 | 20,8 | 72 | 184 | 21,2 | – | 95,0 | – | 80 | 40 |
| A | 22,2 | 72 | 184 | 0 | 189 | 92,6 | 3,4 | 216 | 36,4 |
| B | – | – | – | – | – | – | – | – | – |
| C | 22,2 | 72 | 184 | 21,2 | | 95 | – | 138 | 14 |

Co : concentration des monomères dans le milieu réactionnel exprimé en pourcentage pondéral.

TxN : Taux de neutralisation de l'acide acrylique exprimé en pourcentage molaire.

TxR : Taux de monomère réticulant exprimé en mmoles pour 1000 moles de monomères.

Cs : Concentration de la silice dans le produit final exprimée en pourcentage pondéral.

Pds : Poids du produit isolé exprimé en grammes.

E.S. : Extrait sec du produit isolé exprimé en pourcentage pondéral.

$H_2O$ : Poids d'eau éliminé par gramme de produit final exprimé en grammes.

L'examen du tableau I montre que les produits de la présente invention présentent des propriétés d'absorption d'eau et d'eau salée identiques ou même supérieures au même produit exempt de silice. En conséquence, pour un poids identique de polymère, les produits de la présente invention possèdent des capacités d'absorption d'eau et d'eau salée nettement améliorées.

## Revendications

1. Superabsorbants en poudre, hydrophiles, insolubles dans l'eau, caractérisés par le fait qu'ils contiennent pondéralement, à l'état sec, en combinaison, de 1 à 45% de silice colloïdale à l'état de particules non agglomérées d'un diamètre moyen compris entre 9 et 50 nm et de 99 à 55% d'un polymère réticulé à base d'acide acrylique libre, partiellement ou totalement salifié par du sodium ou du potassium.

2. Superabsorbants selon la revendication 1 caractérisés par le fait qu'ils contiennent pondéralement à l'état sec, de 10 à 40 % de silice et 90 à 60 % d'un polymère réticulé acide acrylique-acrylate de sodium ou de potassium contenant en proportions molaires de 10 à 50 % de motifs acide acrylique.

3. Superabsorbants selon l'une quelconque des revendications 1 et 2 caractérisés par le fait qu'ils contiennent à l'état sec, de 10 à 40 % de silice colloïdale à l'état de particules non agglomérées d'un diamètre moyen compris entre 20 et 50 nm et 90 à 60 % d'un copolymère réticulé acide acrylique-acrylate de sodium ou de potassium contenant en proportions molaires de 15 à 35 % de motifs acide acrylique.

4. Procédé de préparation des superabsorbants selon l'une quelconque des revendications 1 à 3 caractérisé par le fait que l'on polymérise dans l'eau, en mélange, la silice colloïdale et l'acide acrylique libre, partiellement ou totalement salifié par du sodium ou du potassium, en présence d'un monomère réticulant hydrosoluble et d'un amorceur de polymérisation hydrosoluble, puis que l'on sèche le gel polymére gonflé d'eau ainsi obtenu jusqu'à une teneur en eau inférieure à 10 % en poids et qu'enfin l'on broit en poudre le gel polymère sec.

5. Procédé selon la revendication 4 caractérisé par le fait que le monomère réticulant est l'acide bisacrylamidoacétique .

6. Procédé selon la revendication 4 caractérisé par le fait que le monomère réticulant est l'acide diallyloxyacétique.

7. Procédé selon l'une quelconque des revendications 4 à 6 caractérisé par le fait que la polymérisation est effectuée de manière quasi adiabatique.

8. Application des superabsorbant tels que définis dans l'une quelconque des revendications 1 à 3 comme agents absorbants de fluides aqueux.

9. Application selon la revendication 8 à l'absorption de fluides aqueux contenant en solutions des sels minéraux.

10. Application selon la revendication 8 ou 9 à l'absorption d'eau de mer.

Office européen
des brevets

# RAPPORT DE RECHERCHE EUROPEENNE

Numero de la demande

EP    91 40 1806

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | WORLD PATENTS INDEX LATEST<br>Derwent Publications Ltd., London, GB;<br>AN 88-288718<br>& JP-A-63 210 109 (KANAE KAGAKU KOGYO) 31 Août 1988<br>* abrégé * | 1-6 | C08K3/36<br>C08F2/44<br>C08F20/26<br>A61L15/00<br>C08L33/02 |
| A | WORLD PATENTS INDEX LATEST<br>Derwent Publications Ltd., London, GB;<br>AN 85-253475<br>& JP-A-60 168 536 (SHINAGAWA FIRE BRICK) 2 Septembre 1985<br>* abrégé * | 1-6 | |
| A | PATENT ABSTRACTS OF JAPAN<br>vol. 8, no. 19 (C-207)(1456) 26 Janvier 1984 (<br>YAMANI YAKUHIN K.K. ) 31 Octobre 1983<br>& JP-A-58 186 437<br>* abrégé * | 1-6 | |

---

**DOMAINES TECHNIQUES RECHERCHES (Int. Cl.5 )**

C08K
C08F
A61L
C08L

Le présent rapport a été établi pour toutes les revendications

| Lieu de la recherche | Date d'achèvement de la recherche | Examinateur |
|---|---|---|
| LA HAYE | 07 NOVEMBRE 1991 | SIEMENS T. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)